# EUROPÄISCHE PATENTSCHRIFT

(11) **EP 1 890 599 B1**
(45) Veröffentlichungstag und Bekanntmachung des Hinweises auf die Patenterteilung: **29.07.2009**
(21) Anmeldenummer: 06754023.7
(22) Anmeldetag: 01.06.2006
(51) Int. Cl.: A61B 5/053, A61B 5/103

(54) **ANORDNUNG UND VERFAHREN ZUR MESSUNG PHYSIOLOGISCHER MESSGRÖSSEN**
ARRANGEMENT AND METHOD FOR MEASURING PHYSIOLOGICAL MEASURED QUANTITIES
ENSEMBLE ET PROCEDE POUR MESURER DES GRANDEURS A MESURER PHYSIOLOGIQUES

(30) Priorität: 01.06.2005 DE 102005025584
(43) Veröffentlichungstag der Anmeldung: 27.02.2008
(73) Patentinhaber: Fraunhofer-Gesellschaft zur Förderung der angewandten Forschung e.V., 80686 München (DE)
(72) Erfinder: PETER, Christian, 18059 Rostock (DE); EBERT, Eric, 18184 Roggentin OT Kösterbeck (DE); SCHUBERT, Kay, 10245 Berlin (DE)
(74) Vertreter: Brunotte, Joachim Wilhelm Eberhard
(86) Internationale Anmeldenummer: PCT/EP2006/005209
(87) Internationale Veröffentlichungsnummer: WO 2006/128697

(56) Entgegenhaltungen:
- EP-A2- 1 102 199
- WO-A-20/05092177
- WO-A1-00/77659
- WO-A2-20/04034881
- US-A- 5 267 568
- US-A1- 2004 133 120
- US-A1- 2004 138 540
- US-A1- 2004 243 328
- US-A1- 2005 033 128
- US-B1- 6 254 536

## Beschreibung

Die Erfindung betrifft eine Anordnung und ein Verfahren zur Messung physiologischer Messgrößen, insbesondere einer Hauttemperatur und/oder eines elektrischen Hautwiderstandes.

Physiologische Messgrößen von Menschen und Tieren werden vielfach beispielsweise in der Psychologie, in der wissenschaftlichen Physiologie, für medizinische Untersuchungen, Überwachungen und Therapien, in der Emotionsforschung, für sportwissenschaftliche Untersuchungen, für Belastungstests, im Arbeitsschutz, für ergonomische Untersuchungen, für die Überwachung und Assistenz von Personen in Fahrzeugen und sicherheitskritische Umgebungen, an Informations-Schnittstellen zwischen Mensch und Maschine, für das so genannte Affective Computing, für Medien, die Emotionen oder Interessen berücksichtigen, für Spiele, für Geräte und/oder für Systeme benötigt.

Physiologische Messgrößen zeichnen sich insbesondere bei den genannten Anwendungsgebieten gegenüber anderen Messgrößen durch einen hohen Bedarf an Zuverlässigkeit des Messverfahrens aus. Insbesondere in medizinischen und sicherheitskritischen Anwendungen wie die Überwachung von Menschen bei sicherheitskritischen Tätigkeiten können Messfehler zu falschen Diagnosen führen und teilweise fatale Auswirkungen haben. Außerdem werden für einige Anwendungen kontinuierlich oder quasi-kontinuierlich aktuelle Messdaten verlangt, etwa für die Steuerung von Maschinen auf Grund von physiologischen Messgrößen, aber auch bei der Überwachung des Körperzustandes von Personen.

Erschwerend kommt hinzu, dass physiologische Messgrößen einer Person häufig während der Bewegung gemessen werden. Einerseits kann sich hierdurch der Zustand der Umgebung ändern und den Zustand der Messgröße verändern. Andererseits besteht die Gefahr, dass der Mess-Sensor relativ zum Körper der Personen bewegt wird, z. B. auf der Haut verschoben wird. Messwerte sind daher verhältnismäßig häufig fehlerhaft und Schwankungen durch externe Einflüsse unterlegen.

Es ist möglich, zur Erhöhung der Zuverlässigkeit der Messwerte redundante Sensoren zu verwenden. Allerdings besteht dabei häufig das Problem, dass die Sensoren an verschiedenen Orten platziert sind und daher unterschiedliche Messwerte liefern bzw. in unterschiedlicher Weise von äußeren Einflüssen abhängen. Sind die redundanten Sensoren dagegen zu nahe beieinander angeordnet, können sie sich gegenseitig stören, insbesondere durch elektrostatische und elektrodynamische Effekte.
US2004138540 (Clark R.Baker JR, 15.07.2004) beschreibt ein Sensorsystem und Messverfahren zur Messung u.a. der Temperatur. Dabei wird dem Messwert eine Qualitätsmatrix zugewiesen. Diese besteht u.a. aus einem Qualitätswert, der die Genauigkeit des gemessenen Wertes angibt. Werte der Qualitätsmatrix werden mittels eines festgelegten Wertebereichs geprüft und darauf basierend entschieden, ob der Messwert angezeigt wird oder nicht. Zusätzlich werden Werte der Qualitätsmatrix angezeigt. Insbesondere wenn der Kontakt des Sensors zur Haut unterbrochen wird, würde das System aus US2004138540 einen unrealistischen, den alten oder gar keinen Wert angeben.

Es ist eine Aufgabe der vorliegenden Erfindung, ein Verfahren und eine Anordnung zur Messung physiologischer Messgrößen anzugeben, die für eine Weiterverarbeitung in den zuvor genannten Anwendungen, insbesondere medizinischen und/oder sicherheitskritischen Anwendungen sowie Affective Computing, geeignete Messwerte liefern. Insbesondere können diese Anwendungen eine hohe Zuverlässigkeit der an sie übertragenen Messergebnisse und eine kontinuierliche Lieferung von aktuellen Messergebnissen verlangen.

Es wird vorgeschlagen, zumindest für einen Teil der Messwerte, vorzugsweise aber für jeden der Messwerte, eine Unsicherheit des Messwertes anzugeben. Durch die Unsicherheit ist definiert, wie weit ein tatsächlicher Wert der physiologischen Messgrößen von dem Messwert abweichen kann. Der durch die

Unsicherheit definierte Wertebereich kann sich bezüglich dem Messwert z. B. symmetrisch zu größeren und kleineren Werten erstrecken. Die Erfindung ist jedoch nicht hierauf beschränkt. Insbesondere kann eine erste Unsicherheit für einen maximalen Fehler zu größeren Werten definiert sein und eine zweite Unsicherheit für einen maximalen Fehler zu kleineren Werten definiert sein.

Außerdem wird vorgeschlagen, die Unsicherheit abhängig von einer Fehler-Überprüfung des Messwertes anzugeben. Ist beispielsweise ein Messwert fehlerhaft (etwa weil er außerhalb eines erwarteten Wertebereichs liegt), wird eine höhere Unsicherheit angegeben als bei einem vorangegangenen nicht fehlerhaften Messwert. Bei dem vorangegangenen Messwert kann es sich um einen in einer Folge von Messwerten unmittelbar vorangegangenen Messwert oder um einen noch früheren Messwert handeln. Umgekehrt kann die Unsicherheit wieder graduell erniedrigt werden oder auf einen Mindestwert zurückgesetzt werden, wenn bei einem neuen Messwert oder bei einer Folge von neuen Messwerten kein Fehler festgestellt wird.

Die Feststellung eines Messwert-Fehlers kann insbesondere abhängig von einem Umgebungszustand sein. Beispielsweise beeinflusst die Umgebungstemperatur sowohl die Hauttemperatur als auch den Hautwiderstand (auf Grund einer veränderten Schweißdrüsenaktivität) beim Menschen. Wird die Umgebungstemperatur miterfasst, kann z. B. ein vordefinierter erwarteter Messwerte-Bereich an eine veränderte Umgebungstemperatur angepasst werden, etwa entsprechend verschoben, vergrößert oder verkleinert werden.

Insbesondere wird ein Verfahren zur Messung physiologischer Messgrößen vorgeschlagen, insbesondere einer Hauttemperatur und/oder eines elektrischen Hautwiderstandes, wobei
- ein Messwert einer physiologischen Messgröße von einem Sensor erfasst wird,
- eine Unsicherheit des Messwertes definiert wird, um die ein tatsächlicher Wert der physiologischen Messgröße von dem Messwert abweichen kann,
- der Messwert in einer Überprüfung auf Fehler überprüft wird und
- die Unsicherheit abhängig von einem Ergebnis der Überprüfung und/oder abhängig von Umgebungsvariablen vergrößert wird, beibehalten wird oder verkleinert wird.

Die Unsicherheit kann jeweils einem Messwert zugeordnet werden. Insbesondere kann die Information über die Unsicherheit dem Messwert zugeordnet ausgegeben werden.

Durch die zusätzliche Information über die Unsicherheit, die beispielsweise bei der Ausgabe der Messergebnisse an einer Auswertungseinrichtung jeweils einem Messwert zugeordnet ist, kann die jeweilige Auswertung physiologischer Messwerte zuverlässiger durchgeführt werden. Ferner wird vorzugsweise auch die explizite Information darüber angegeben und/oder ausgegeben, ob ein Messwert fehlerhaft ist. Außerdem kann eine Fehlerkategorie und/oder ein Grad eines Fehlers angegeben und/oder ausgegeben werden.

Auf Basis der zusätzlichen Information können verschiedene Maßnahmen ergriffen werden oder unterlassen werden. Beispielsweise können unsichere Messwerte durch vorangegangene sicherere Messwerte ersetzt werden und/oder erst dann die Auswertung unterbrochen oder ausgesetzt werden, wenn die Unsicherheit einen Unsicherheits-Grenzwert erreicht oder überschritten hat. Alternativ oder zusätzlich können Auswertungsverfahren abhängig von der zeitlichen Entwicklung der Messwerte und ihrer Unsicherheit entscheiden, ob bestimmte Aktionen ausgeführt werden müssen, Aktionen unterbleiben müssen und/oder bestimmte Feststellungen getroffen werden müssen. Zum Beispiel kann daher die Entscheidung darüber, ob eine mit Hilfe der physiologischen Messgröße überwachte Person (etwa ein Fluglotse) sicherheitskritische Aufgaben noch zuverlässig erfüllt, auf Grund wesentlich aussagekräftigerer Informationen getroffen werden.

Weiterhin ist es möglich, die Messung der physiologischen Messgröße abhängig von der Größe der Unsicherheit und/oder abhängig von der Information, ob ein Messfehler erkannt wurde oder nicht, zu verändern. Insbesondere können zumindest Teile der Mess-Sensorik (Sensor zuzüglich Messsignal-Aufbereitung) einem Selbsttest unterzogen werden, rekalibriert werden und/oder die Position zumindest eines Sensors (insbesondere relativ zu einem Messobjekt, z. B. relativ zur Hautoberfläche) neu eingestellt werden.

Auch ist eine entsprechende Mess-Anordnung zur Messung der physiologischen Messgröße durch die Angabe der Unsicherheit nahezu universell für Anwendungen geeignet. Die jeweilige Anwendung benötigt nur reduzierte Informationen oder keine Informationen über die Fehlerursachen bei Messwertfehlern. Sie kann sich vielmehr auf die Angabe der Unsicherheit und optional auf die zusätzliche Angabe, in welcher Kategorie gegebenenfalls ein Fehler liegt, verlassen. Es ist auch kein Spezialwissen über die Hardware (Herstellerwissen) von Nöten.

Die Unsicherheit kann abhängig von einer Art eines festgestellten Fehlers weniger stark verändert oder stärker verändert werden. Eine Fehler-Art ist insbesondere ein Messfehler, d.h. ein Fehler bei der Messung der physiologischen Messgröße. In besonderer Ausgestaltung können z. B. Fehler bei der Übertragung der Messwerte von der Mess-Sensorik zu der Messwert-Überprüfungseinrichtung in die von der Überprüfungseinrichtung durchgeführte Fehlerüberprüfung einbezogen werden. Dabei kann es sich um eine andere Fehler-Art handeln, die für die Unsicherheit berücksichtigt wird.

Vorzugsweise werden die Messwerte drahtlos zu der Überprüfungseinrichtung übertragen, sodass dabei häufig Übertragungsfehler auftreten können. Die Überprüfungseinrichtung kann die Messwerte und Zusatzinformationen dann zu einer Auswertungseinrichtung weiterleiten. Beispielsweise wird bei der drahtlosen Übertragung zu der Überprüfungseinrichtung mit Hilfe von Prüfsummen ermittelt, ob ein Übertragungsfehler aufgetreten ist. Stellt die Überprüfungseinrichtung fest, dass ein Übertragungsfehler vorliegt, kann der Fehler anders als ein Messfehler behandelt werden. Insbesondere wird die Unsicherheit bei einem solchen Fehler weniger stark erhöht als bei einem Messfehler. Dies hat den Vorteil, dass häufig auftretende Übertragungsfehler das Ergebnis nicht so stark beeinflussen wie Messfehler und den Auswertungsprozess nicht unnötig stören. Da Übertragungsfehler häufig statistisch über die Zeit verteilt auftreten, erscheint dies gerechtfertigt. Eine drahtlose Übertragung der Messwerte zu der Überprüfungseinrichtung hat den Vorteil, dass sich der Proband frei bewegen kann und dass die eigentlichen Messsignale an einem anderen Ort weiterverarbeitet werden. Die Mess-Sensorik kann daher verhältnismäßig einfach und leicht ausgestaltet werden.

Bei der Fehler-Überprüfung kann auf ein Vorliegen eines Fehlers erkannt werden, wenn der Messwert außerhalb eines vorgegebenen, erwarteten Messwertbereiches liegt. Beispielsweise können sinnvolle Messwerte bei einer Hautwiderstands-Messung nur in einem bestimmten Wertebereich liegen, wobei der Wertebereich optional individuell für jeden Probanden, jede Mess-Situation und/oder jede Mess-Sensorik definiert werden kann. Löst sich der Sensor z. B. auf Grund einer Bewegung des Probanden von dessen Haut, treten Messwerte außerhalb des definierten Wertebereichs auf und es wird auf einen Messfehler erkannt.

Alternativ oder zusätzlich kann bei der Überprüfung auf ein Vorliegen eines Fehlers erkannt werden, wenn der Messwert sich schneller als eine vorgegebene Änderungsrate ändert. Die vorgegebene Änderungsrate wird vorzugsweise unter Berücksichtigung von Kenntnissen über die Physiologie definiert. Auch die Änderungsrate kann optional individuell für jeden Probanden, jede Mess-Situation und/oder jede Mess-Sensorik definiert werden. Zum Beispiel kann sich der Hautwiderstand eines Probanden nicht stärker als mit der vorgegebenen Änderungsrate ändern, da die Produktion von Schweiß begrenzt ist bzw. die Verdunstung von Schweiß begrenzt ist.

Allgemeiner formuliert kann die vorgegebene Änderungsrate von einer zeitlichen Entwicklung des Messwertes abhängig festgelegt werden. Insbesondere kann in diesem Fall die zeitliche Entwicklung der als fehlerfrei festgestellten Messwerte bis zu dem Zeitpunkt des aktuellen Messwertes extrapoliert werden. Beispielsweise kann die vorgegebene Änderungsrate bei größeren Messwerten größer sein als bei kleineren Messwerten.

In einem weiteren Fall, der auch mit dem in dem vorangegangenen Absatz beschriebenen Fall kombiniert werden kann, wird zumindest eine weitere physiologische Messgröße oder eine andere Messgröße bei der Festlegung der vorgegebenen Änderungsrate berücksichtigt (z. B. Hauttemperatur, Hautwiderstand und Umgebungstemperatur werden berücksichtigt). Dabei kann insbesondere ein physikalisches Modell der Messanordnung mit den entsprechenden Messsensoren zur Berechnung der vorgegebenen Änderungsrate verwendet werden.

Mit der Kombination der zwei zuvor definierten Fehlerkriterien (Bereich und Änderungsrate) kann ein Messfehler sehr zuverlässig erkannt werden. Dies ist besonders dann von Vorteil, wenn der Sensor ein nicht invasiver Sensor ist, der an einer Hautoberfläche eines Probanden angeordnet ist. Bewegungen des Probanden führen in diesem Fall wie erwähnt zu häufigen Messfehlern. Auch ist die Kombination der beiden Kriterien in der Praxis mit geringem verarbeitungstechnischem Aufwand anwendbar.

Es kann eine Fehlerklassifikation durchgeführt werden, d.h. bei einem erkannten Fehler eine Information über eine Klasse oder Kategorie des Fehlers generiert werden. Die Klasse oder Kategorie kann bei der weiteren Datenverarbeitung genutzt werden, z. B. bei der Berechnung der Unsicherheit.

Der nicht invasive Sensor kann an einer festen Position relativ zu einer Hautoberfläche eines Probanden fixiert sein. Hierdurch können Schwankungen des von dem Sensor gelieferten Messsignals vermieden werden. Zum Beispiel kann ein unmittelbarer Kontakt zwischen dem Sensor und der Haut eines Probanden erforderlich sein, um einen fehlerfreien Messwert zu liefern. Dabei hängt die Messgröße in der Regel außerdem von dem Ort an der Hautoberfläche des Probanden ab. Wird der Sensor auf der Haut verschoben, liefert er einen anderen Messwert.

Vorzugsweise ist der Sensor daher in oder an einem Formstück befestigt, das wiederum in einer definierten Position relativ zu der Haut angeordnet ist. Dabei kann das Formstück Bewegungen des Probanden erlauben und fixiert den Sensor dennoch an einer festen Position relativ zu der Hautoberfläche. Beispielsweise ist das Formstück oder ein Teil davon aus flexiblem und/oder elastischem Material gefertigt. So kann z. B. ein Handschuh als Formstück verwendet werden, der optional ein elastisches Element aufweist, sodass der Sensor unter Ausnutzung elastischer Kräfte an der Hautoberfläche fixiert wird. Dabei kann je nach Art des Sensors unmittelbarer Hautkontakt bestehen oder es kann sich noch ein anderes Material zwischen dem Sensor und der Haut befinden.

Die Fixierung der Position kann jedoch z. B. auch dadurch erreicht werden, dass die Person das Formstück mit ihrer Hand umgreift. Das Formstück ist in diesem Fall entsprechend ausgestaltet und weist z. B. einen Außenumfang auf, der von einer menschlichen Hand zumindest teilweise umfasst werden kann. Der oder die Sensoren zum Messen der physiologischen Größe(n) sind dabei vorzugsweise so angeordnet, dass sie in Kontakt zu der Haut der Person gelangen, wenn diese das Formstück mit der Hand hält. Das Formstück kann insbesondere eine Steuereinrichtung (z. B. ein Controller mit Bedienelementen zur Steuerung einer technischen Einrichtung, etwa eine Spiel-Konsole und/oder eine Fernbedienung), eine Interaktionseinrichtung (z. B. ein Controller, der auch Signale empfängt und z. B. haptisch ausgibt) und/oder eine Kommunikationseinrichtung (z. B. ein Mobiltelefon zum Betrieb in einem Mobilfunknetz, und/oder ein Gerät zur Überwachung von physiologischen Messgrößen der Person für medizinische Zwecke, z. B. für Risikopatienten und/oder zur Überwachung von Personen mit sicherheitskritischen Aufgaben wie Lokomotivführer oder Fluglotsen) sein.

Die Erfindung erstreckt sich jedoch auch auf andere Arten von Sensoren zur Messung physiologischer Messgrößen, z. B. invasive Sensoren wie implantierte mikroelektronische Sensoren, akustische Sensoren (beispielsweise zur messtechnischen Erfassung von Stimmen und/oder Frequenzverläufen) optische Sensoren (beispielsweise einer Kamera zur Beobachtung und Auswertung der Mimik einer Person).

In oder an dem Formstück kann außer dem Sensor oder den Sensoren auch die Überprüfungseinrichtung zur Überprüfung der Messwerte auf Fehler und optional auch die Bewertungseinrichtung angeordnet sein. Beispielsweise sind sämtliche Einrichtungen, mit Ausnahme der Sensoren, in ein Bauteil integriert, das gemeinsam mit einer Batterie zur Stromversorgung an dem Formstück angebracht sein kann. Das Bauteil mit der Batterie wird vorzugsweise über eine lösbare Klemmverbindung und/oder lösbare Rastverbindung, die gleichzeitig einen mechanischen und einen elektrischen Kontakt zu dem Formstück herstellt, mit dem Formstück verbunden. Für die Verbindung kann beispielsweise ein metallischer Druckknopf verwendet werden, wie er als Verschluss für Bekleidung bekannt ist. Allgemeiner formuliert kann die Verbindung formschlüssig und/oder kraftschlüssig hergestellt werden. Wenn das Formstück ein Handschuh ist, wird das Bauteil beispielsweise am Übergang des Handrückens zum Unterarm einer Person angebracht, die den Handschuh trägt. Das Bauteil kann auch ein Funkmodul zur drahtlosen Übertragung von Daten aufweisen. Eine Sendeantenne kann optional außerhalb des Bauteils angeordnet sein.

Zur elektrischen Verbindung des Sensors oder der Sensoren mit einer Einrichtung zur Verarbeitung der Sensorsignale (auf Beispiele für die Verarbeitung wird noch in der Figurenbeschreibung eingegangen) können Fäden (z. B. Goldfäden mit elektrisch isolierender Ummantelung) aus elektrisch leitendem Material verwendet werden, die in das Formstück eingenäht sind, welches beispielsweise zumindest teilweise durch Textilmaterial (z. B. ein- oder mehrlagiger Webstoff) gebildet wird. Ein oder mehrerer derartige Fäden können auch die Sendeantenne bilden.

Der Sensor kann eine (vorzugsweise flexible, d. h. biegbare) Platine aufweisen, die im Bereich ihres Randes eine Mehrzahl von Löchern aufweist. Die Platine trägt Elektroden eines Hautwiderstands-Sensors und/oder trägt einen Temperatursensor. Durch einen durch die Löcher verlaufenden Faden (oder durch mehrere Fäden) kann die Platine an das Formstück angenäht werden. Dabei kann der Faden oder zumindest einer der Fäden elektrisch leitfähig sein und der elektrischen Verbindung des Sensors mit anderen Einrichtungen dienen.

Insbesondere kann zumindest eine weitere physiologische Messgröße (z. B. die Hauttemperatur) und/oder eine andere Messgröße (z. B. die Umgebungstemperatur) parallel zu der ersten physiologischen Messgröße gemessen werden und ebenfalls die Unsicherheit für diese Messgröße ausgegeben werden. Die Sensoren zum Messen der Messgrößen können dabei an derselben Einrichtung angeordnet sein, z. B. an demselben Formstück (beispielsweise Handschuh). Vorzugsweise werden dann die Unsicherheiten einer Mehrzahl von Messgrößen ausgewertet und es wird festgestellt, ob ein Fehler der gesamten Messeanordnung vorliegt, z. B. das Formstück nicht korrekt an der Haut des Probanden anliegt. Diese Auswertung kann durch eine Einrichtung durchgeführt werden, die Teil der Bewertungseinrichtung ist, die in oder an dem Formstück angeordnet ist und/oder die Teil einer nachgeschalteten Auswertungseinrichtung ist, die die Messwerte und die Unsicherheiten empfängt.

Es können weitere Fehlerkriterien definiert werden, die alternativ oder zusätzlich angewendet werden. An Stelle einer fest vorgegebenen Änderungsrate kann beispielsweise ein physikalisches Modell der Mess-Situation verwendet werden, sodass ein Vergleich mit einem von dem Modell vorausgesagten Messwert und dem tatsächlichen Messwert als Kriterium dienen kann.

Insbesondere wenn das beschriebene Kriterium der Änderungsrate angewendet wird und eine Fehler-Überprüfung für eine Folge von Messwerten des Sensors durchgeführt wird, kann zumindest ein vorhergehender Messwert der Folge gespeichert werden. Durch einen Vergleich des gespeicherten Messwertes mit einem folgenden, zweiten Messwert kann dann eine Änderungsrate zum Vergleich mit der vorgegebenen Änderungsrate bestimmt werden. Die Speicherung einer Mehrzahl der vorhergehenden Messwerte hat den Vorteil, dass ein Vergleichswert auch dann für die Fehler-Überprüfung zur Verfügung steht, wenn lediglich einer der gespeicherten Messwerte fehlerfrei ist. Insbesondere für diese Ausgestaltung wird daher bevorzugt, dass der Fehlerzustand ("fehlerfrei" oder "Fehler besteht", bzw. Fehlerkategorie) explizit mit abgespeichert wird. Die Bestimmung der aktuellen Änderungsrate wird vorzugsweise unter Verwendung eines fehlerfreien gespeicherten Messwertes durchgeführt, der in der Folge der Messwerte dem zu überprüfenden Messwert unmittelbar vorher geht. Alternativ (insbesondere für den Fall, dass der Messwert nicht fehlerfrei ist) kann die aktuelle Änderungsrate auch unter Verwendung früherer gespeicherter Messwerte ermittelt werden.

Bei einer bevorzugten Ausgestaltung des Verfahrens wird die Unsicherheit eines späteren Messwertes gegenüber der Unsicherheit eines früheren Messwertes um einen Betrag erhöht, der abhängig von der vorgegebenen Änderungsrate und abhängig von einer Zeitdifferenz zwischen dem Messzeitpunkt des späteren Messwertes und dem Messzeitpunkt des früheren Messwertes ist, insbesondere gleich der vorgegebenen Änderungsrate multipliziert mit der Zeitdifferenz ist. Somit ist sichergestellt, dass die Unsicherheit beim Auftreten von Messfehlern nicht zu schnell anwächst.

Vorzugsweise wird kontinuierlich ein validierter Messwert ausgegeben, wobei der validierte Messwert gleich dem Messwert ist, wenn bei der Überprüfung des Messwertes kein Fehler festgestellt wird, und wobei der validierte Messwert durch Korrektur des Messwertes erzeugt wird, wenn bei der Überprüfung des Messwertes ein Fehler festgestellt wird. Unter kontinuierlicher Ausgabe wird auch die quasi-kontinuierliche Ausgabe von z. B. digitalen Messwerten zu diskreten Zeitpunkten verstanden, bei der mit vorzugsweise gleich bleibenden Zeitabständen eine zeitliche Folge der validierten Messwerte ausgegeben wird. Die kontinuierliche Ausgabe eines validierten Messwertes hat den Vorteil, dass die jeweilige Anwendung, die die validierten Messwerte verwendet, kontinuierlich auf aktuelle Messwerte zurückgreifen kann, die außerdem gegebenenfalls korrigiert wurden. Daher kann die Anwendung mit hoher Zuverlässigkeit arbeiten. In der Anwendung müssen keine Vorkehrungen für den ansonsten häufig auftretenden Fall getroffen werden, dass Messwerte vorübergehend ausbleiben.

Wenn ein Messwert als fehlerhaft erkannt worden ist, kann die Korrektur auf unterschiedliche Weise durchgeführt werden. In einem bevorzugten Fall, der sich durch geringen Rechenaufwand auszeichnet, wird der zuletzt als fehlerfrei festgestellte Messwert als validierter Messwert ausgegeben.

In einer anderen Ausgestaltung wird zur Korrektur eine zeitliche Entwicklung des Messwertes berücksichtigt. Insbesondere kann in diesem Fall aus der zeitlichen Entwicklung der als fehlerfrei festgestellten Messwerte bis zu dem Zeitpunkt des aktuellen, als fehlerhaft erkannten Messwertes extrapoliert werden. Dabei ist es optional möglich, das Ergebnis der Extrapolation noch zu verändern. Beispielsweise kann ein sich aus der zeitlichen Entwicklung ergebender Anstieg des Messwertes verringert werden, d. h. es ergibt sich ein korrigierter Messwert, der zwischen dem zuletzt als fehlerfrei erkannten Messwert und dem extrapolierten Messwert liegt.

In einer weiteren Ausgestaltung, die auch mit dem in dem vorangegangenen Absatz beschriebenen Fall kombiniert werden kann, wird der Messwert zumindest einer weiteren physiologischen Messgröße oder einer anderen Messgröße bei der Korrektur berücksichtigt (z. B. gemessener Hauttemperatur, Hautwiderstand und Umgebungstemperatur werden gegenseitig jeweils bei der Korrektur berücksichtigt). Dabei kann insbesondere ein physikalisches Modell der Messanordnung mit den entsprechenden Messsensoren zur Berechnung der Korrektur verwendet werden.

Alternativ oder zusätzlich können die Verfahren der beiden zuvor genannten Ausgestaltungen der Fehlerkorrektur auch bei der Überprüfung, ob ein Messwert fehlerhaft ist, angewendet werden. So kann z. B. durch Extrapolation des zeitlichen Verlaufs der fehlerfreien Messwerte festgestellt werden, ob der aktuelle Messwert plausibel ist, und/oder kann zumindest eine weitere Messgröße bei der Überprüfung auf Fehler berücksichtigt werden.

Die folgende Ausgestaltung ist unabhängig davon, ob eine Änderungsrate ermittelt wird und als Fehlerkriterium verwendet wird. Wiederum wird die Fehler-Überprüfung für eine Folge von Messwerten des Sensors durchgeführt. Die Ausgestaltung betrifft jedoch unmittelbar die Bestimmung der aktuellen Unsicherheit. Es wird jeweils die Unsicherheit von einem vorhergehenden Messwert der Folge (vorhergehende Unsicherheit) bei einem folgenden, zweiten Messwert verwendet. Nach der Überprüfung des zweiten Messwertes wird die vorhergehende Unsicherheit abhängig von dem Ergebnis der Überprüfung beibehalten oder verändert und die beibehaltene oder veränderte Unsicherheit wird dem zweiten Messwert zugeordnet. Bei dem vorhergehenden Messwert handelt es sich vorzugsweise um den in der Folge der Messwerte unmittelbar vorhergehenden Messwert.

Auf diese Weise kann die Unsicherheit kontinuierlich an den jeweils aktuellen Messwert angepasst werden. Dabei muss allerdings nicht zwangsläufig jedem der Messwerte eine Unsicherheit zugeordnet werden. Zum Beispiel bei einer hohen Messfrequenz kann auf die Auswertung oder Bewertung eines Teils der Messwerte verzichtet werden. Außerdem kann die Anpassung der Unsicherheit dann entfallen, wenn die Unsicherheit einen vorgegebenen Maximalwert erreicht hat und/oder schon über einen längeren Zeitraum hinweg (dessen Länge vorher festgelegt werden kann) kontinuierlich Messfehler vorliegen, sodass eine Verwendung der Messwerte nicht mehr sinnvoll erscheint und auch die Unsicherheit keine sinnvoll verwertbare Zusatzinformationen mehr darstellt.

Die Erfindung betrifft außerdem eine Anordnung zur Messung physiologischer Messgrößen, insbesondere einer Hauttemperatur und/oder eines elektrischen Hautwiderstandes. Die Anordnung kann ausgestaltet sein, das erfindungsgemäße Verfahren in einer oder mehrerer Ausgestaltungen, die in dieser Beschreibung beschrieben sind, durchzuführen. Insbesondere kann die Anordnung Folgendes aufweisen:
- einen Sensor, der ausgestaltet ist, Messwerte einer physiologischen Messgröße zu messen,
- eine Messwert-Überprüfungseinrichtung, die ausgestaltet ist, einen Messwert auf Fehler zu überprüfen,
- eine Bewertungseinrichtung, die ausgestaltet ist, eine Unsicherheit für Messwerte abhängig von einem Ergebnis einer Überprüfung der Messwert-Überprüfungseinrichtung zu vergrößern, beizubehalten oder zu verkleinern, wobei die Unsicherheit des Messwertes angibt, wie weit ein tatsächlicher Wert der physiologischen Messgröße von dem Messwert abweichen kann.

Außerdem kann die Anordnung z. B. ausgestaltet sein, kontinuierlich einen validierten Messwert auszugeben, wobei der validierte Messwert gleich dem Messwert ist, wenn bei der Überprüfung des Messwertes kein Fehler festgestellt wird, und wobei der validierte Messwert ein durch Korrektur des Messwertes erzeugter Wert ist, wenn bei der Überprüfung des Messwertes ein Fehler festgestellt wird.

Weiterhin kann die Überprüfungseinrichtung ausgestaltet sein, bei der Überprüfung eine Plausibilitätsprüfung des Messwertes durchzuführen, insbesondere auf ein Vorliegen eines Fehlers zu erkennen,
- wenn der Messwert außerhalb eines vorgegebenen, erwarteten Messwertbereiches liegt,
- wenn die Messgröße sich schneller oder langsamer als mit einer vorgegebenen Änderungsrate ändert und/oder
- wenn der Messwert nicht einem Wert entspricht, der unter Verwendung eines physikalischen Modells der Messanordnung berechnet wurde, wobei z. B. ein Computer die Modellrechnung durchführt.

Beispielsweise kann ein mit der Messanordnung verbundener (z. B. in das Formstück integrierter) Schrittzähler bei der Plausibilitätsprüfung zu dem Zwischenergebnis führen, dass die überwachte Person ein höheres Lauftempo als zuvor hat. Daraus kann geschlossen werden, dass eine höhere Herzschlagfrequenz erwartet wird. Tritt die höhere Herzschlagfrequenz nicht oder nicht schnell genug ein, kann auf das Vorliegen eines Fehlers geschlossen werden. Dies ist ein Fall gemäß oben genannter Alternative, wonach sich eine Messgröße zu langsam ändert. Die Erwartung der höheren Herzschlagfrequenz kann mit Hilfe des physikalischen Modells der Messanordnung ermittelt werden, wobei das Körperverhalten der Person im Modell berücksichtigt ist. "Physikalisches Modell" schließt daher ganz allgemein auch das Körperverhalten (insbesondere Zusammenhang zwischen Belastung und Herzschlagfrequenz bzw. Pulsfrequenz sowie Hauttemperatur) mit ein.

Die Erfindung wird nun unter Bezugnahme auf die beigefügte Zeichnung anhand von Ausführungsbeispielen näher beschrieben. Sie ist jedoch nicht auf die Ausführungsbeispiele beschränkt. Einzelne technische Merkmale der Ausführungsbeispiele oder beliebige Kombinationen davon können mit den zuvor beschriebenen Ausgestaltungen der Erfindung kombiniert werden. Die einzelnen Figuren der Zeichnung zeigen:
- Fig. 1: eine Anordnung zur Messung und Auswertung einer physiologischen Messgröße,
- Fig. 2: ein Diagramm, das schematisch Rohdaten einer Messung, eine daraus erzeugte Ausgangsgröße und die Unsicherheit der Ausgangsgröße jeweils als Funktion der Zeit darstellt.

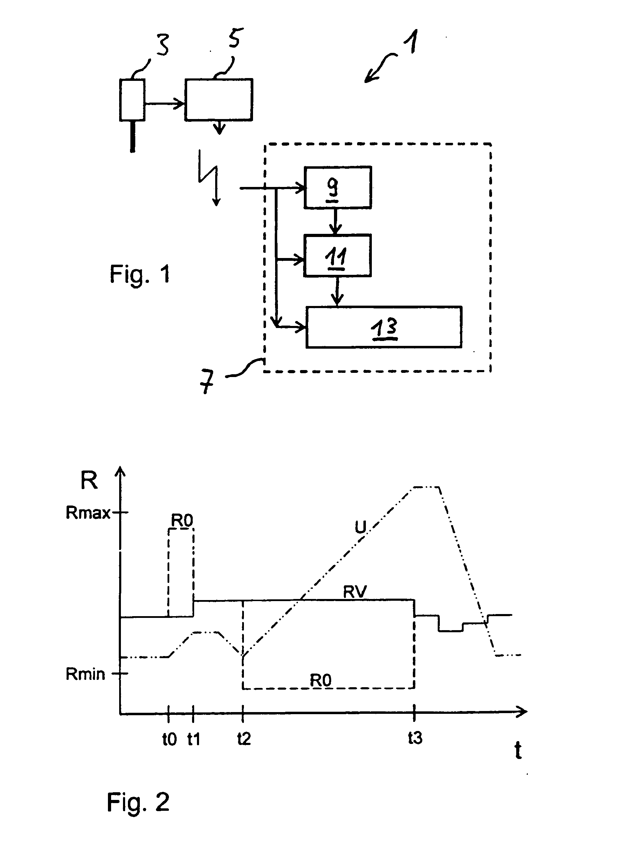

Die in Fig. 1 schematisch dargestellte Anordnung 1 weist einen Sensor 3 auf, der in dem Beispiel ein Widerstandssensor zur Messung eines Hautwiderstandes ist. Beispielsweise weist der Hautwiderstandssensor ein Elektrodenpaar auf, das an der Hautoberfläche eines Probanden angeordnet werden kann. Mit dem Sensor 3 ist eine Verarbeitungseinrichtung 5 verbunden, die das kontinuierlich erzeugte Messsignal des Sensors 3 digitalisiert und zur weiteren Verarbeitung über eine geeignete Schnittstelle (z.B. Funk) zu einer Recheneinrichtung 7 überträgt. Die Funkschnittstelle ist in Fig. 1 durch einen gezackten Pfeil dargestellt. Der Sensor 3 und die Verarbeitungseinrichtung 5 bilden zusammen die Mess-Sensorik, die noch weitere Sensoren aufweisen kann, beispielsweise einen weiteren Hautwiderstandssensor, einen Sensor zur Bestimmung der Herzfrequenz des Probanden und/oder einen oder mehrere Temperatursensoren zur Bestimmung der Hauttemperatur und der Umgebungstemperatur.

Von der Recheneinrichtung 7, die beispielsweise digitale Speicher und mikroelektronische Bauelemente zur Verarbeitung der über die Funkschnittstelle empfangenen digitalen Daten aufweist, werden die Funksignale zunächst in an sich bekannter Weise durch nicht dargestellte Empfangseinheiten aufbereitet und in digitale Daten umgewandelt. Die digitalen Daten werden einer Überprüfungseinrichtung 9 zur Überprüfung der Messwerte des Sensors 3, einer Bewertungseinrichtung 11 zur Ausgabe einer Unsicherheit des Messwertes und einer Auswertungseinrichtung 13 zur Auswertung der physiologischen Messgröße zugeführt. Dabei kann beispielsweise ein Datenbus vorgesehen sein.

Die Überprüfungseinrichtung 9 ist mit der Bewertungseinrichtung 11 verbunden, so dass die Bewertungseinrichtung 11 die Unsicherheit abhängig von einem Ergebnis der Überprüfung der Messwerte durch die Überprüfungseinrichtung 9 berechnen kann. Es können weitere nicht in Fig. 1 dargestellte Komponenten der Recheneinrichtung 7 vorgesehen sein, insbesondere ein Speicher zur Speicherung der digitalen Daten, auf den die Einheiten 9, 11 und 13 Zugriff haben. Insbesondere können diese Einheiten in Hardware und/oder Software ausgeführt sein.

Die Auswertungseinrichtung 13 ist mit der Bewertungseinrichtung 11 verbunden und wertet die physiologische Messgröße auf Basis der berechneten Unsicherheit und auf Basis der zugeordneten Messwerte aus. Außerdem stellt die Überprüfungseinrichtung 9 oder eine andere Einrichtung der Recheneinrichtung 7 fest, in welcher Fehlerkategorie der jeweilige auf Fehler zu überprüfende Messwert liegt. Beispielsweise lautet eine der Fehlerkategorien "fehlerfrei", eine andere "Fehler möglich", eine weitere Kategorie "Fehler erkannt" und eine noch weitere Kategorie "Messung zur Zeit falsch". Dabei wird vorzugsweise eine Historie zurückliegender Messwerte ausgewertet. Dadurch kann zuverlässig festgestellt werden, ob nur ein einzelner Messwert falsch ist (beispielsweise durch eine Störung der Funkübertragung oder weil ein einzelner Ausreißer vom Sensor 3 produziert wurde) oder ob zurzeit dauerhaft falsche Messwerte zu erwarten sind.

Wie bereits erwähnt, handelt es sich bei der anhand von Fig. 1 beschriebenen Anordnung lediglich um ein Ausführungsbeispiel. Es können diverse Modifikationen vorgenommen werden. Unabhängig von dem konkreten Ausführungsbeispiel der Fig. 1, kann z. B. in und/oder an dem Formstück, das den oder die Sensoren trägt, eine Einrichtung angeordnet sein, die die Sensorsignale zwar weiterverarbeitet, aber nicht die Überprüfungseinrichtung und nicht die Bewertungseinrichtung aufweist. Diese Einrichtung kann z. B. die Sensorsignale in Daten umwandeln, die auf physikalische Dimensionen wie elektrischer Widerstand und Temperatur bezogen sind, und/oder analoge Sensorsignale digitalisieren.

Alternativ oder zusätzlich kann diese Einrichtung prüfen, ob das Messsignal einem Messwert entspricht, der in einem physikalisch sinnvollen Bereich liegt, und/oder ob das Verhalten (insbesondere das zeitliche Verhalten) des Sensors oder der Sensoren auf einen Fehler schließen lässt. Wenn dies nicht der Fall ist, kann auf das Vorliegen eines Fehlers erkannt werden und eine entsprechende Information an die Überprüfungseinrichtung weitergeleitet werden, die dann den Messwert als fehlerhaft behandelt. Alternativ kann bereits die Einrichtung, die die Sensorsignale weiterverarbeitet, den fehlerhaften Messwert korrigieren, z. B. durch einen älteren Messwert ersetzen und vorzugsweise auch eine Information über das Vorliegen eines älteren Messwertes an die Überprüfungseinrichtung weiterleiten. Ferner kann diese Einrichtung z. B. CRC-Prüfsummen für die (insbesondere drahtlose) Übertragung an die Überprüfungseinrichtung berechnen (siehe auch oben zu den Prüfsummen). Eine oder mehrere dieser Funktionen können aber alternativ von der Überprüfungseinrichtung oder einem Teil der Überprüfungseinrichtung ausgeübt werden, die bzw. der in oder an dem Formstück vorgesehen sein kann oder von dem Formstück entfernt (z. B. über eine Funkschnittstelle mit dieser verbunden) angeordnet sein kann.

Die Überprüfungseinrichtung und die Bewertungseinrichtung können separat von dem Formstück angeordnet sein, und z. B. über eine Funkschnittstelle zur drahtlosen Übertragung (zum Beispiel gemäß dem Bluetooth-Standard) von Daten mit dem Formstück verbunden sein. Die Überprüfungseinrichtung und optional auch die Bewertungseinrichtung können jedoch bei einer besonders bevorzugten Ausführungsform auch in und/oder an dem Formstück angeordnet sein. Das Formstück ist insbesondere ein Handschuh. In diesem Fall kann ein Nutzer die Messdaten empfangen und ohne Expertenwissen auswerten. Er benötigt außer dem Formstück lediglich z. B. noch einen handelsüblichen PC mit geeigneter allgemein erhältlicher Software.

Alternativ kann jedoch auch Software verwendet werden, die das Expertenwissen enthält, und insbesondere die Überprüfungseinrichtung und/oder die Bewertungseinrichtung realisiert.

Die Überprüfungseinrichtung kann so ausgestaltet sein, dass sie eine Plausibilitätsprüfung der Messwerte in dem bereits beschriebenen Sinn durchführt und dem jeweiligen Messwert eine Information zuordnet (beispielsweise ein Flag setzt), die aussagt, ob und/oder in welchem Maße der Messwert fehlerhaft ist. Dabei kann die Information optional auch eine zusätzliche Fehler-Information enthalten, beispielsweise um welche Art von Fehler es sich handelt (z. B. dass es sich um einen früheren Messwert handelt, der als validierter Messwert in dem oben beschriebenen Sinn an Stelle des eigentlichen Messwertes weitergeleitet wird) und/oder wie gravierend der Fehler ist. Allgemeiner formuliert kann die Überprüfungseinrichtung optional auch die Funktion ausführen, wonach in der oben beschriebenen Weise ein validierter Messwert erzeugt wird. Insbesondere kann dieser validierte Messwert der Messwert sein, dem die Information über den Fehlerzustand zugeordnet wird.

Die Bewertungseinrichtung kann dann so ausgestaltet sein, dass sie lediglich anhand der zugeordneten Information feststellt, wie mit der Unsicherheitsangabe zu Verfahren ist, insbesondere ob die Unsicherheit des zugeordneten Messwertes gegenüber einer für vorangegangene Messwerte gültigen Unsicherheit erhöht oder erniedrigt werden muss.

Generell, auch bei anderen Ausführungsformen der Erfindung, können einzelne Messfehler leicht dadurch korrigiert werden, dass als Ausgangssignal der Messwertüberprüfung der zuletzt als fehlerfrei erkannte Messwert ausgegeben wird. Dies gilt mit einer Modifikation, auf die noch näher eingegangen wird, auch für Phasen länger anhaltender Messfehler. Die überprüften und gegebenenfalls korrigierten Messwerte werden als Ergebnis der Überprüfung an die Auswertungseinrichtung, insbesondere an die Auswertungseinrichtung (13) gemäß Fig. 1, übergeben, die beispielsweise aus dem Hautwiderstand und der Umgebungstemperatur ermittelt, ob ein Proband ungewöhnlich auf eine Situation reagiert. Weitere Anwendungsmöglichkeiten sind an sich bekannt und wurden eingangs erwähnt.

Da der Auswertungseinrichtung die von der Bewertungseinrichtung ermittelte Unsicherheit als Zusatzinformation zur Verfügung steht, kann sich die Auswertungseinrichtung auf die eigentliche Auswertung konzentrieren. Insbesondere muss in der Auswertungseinrichtung selbst nicht Expertenwissen darüber implementiert sein, wie mit einem sich schlagartig ändernden Messsignal umzugehen ist, oder darüber, wie zuverlässig ein älterer Messwert zum gegenwärtigen Zeitpunkt noch bei der Auswertung verwendet werden kann. Daher kann die Auswertungseinrichtung leicht durch eine andere
Auswertungseinrichtung ersetzt werden, die die physiologischen Messwerte auswertet. Außerdem kann die Auswertungseinrichtung separat von der Überprüfungseinrichtung und separat von der Bewertungseinrichtung realisiert werden. Insbesondere können die Überprüfungseinrichtung und die Bewertungseinrichtung Teil einer gemeinsamen gerätetechnischen Einheit sein (z.B. Platine, bestückt mit mikroelektronischen Bauelementen) und kann die Auswertungseinrichtung als Teil eines handelsüblichen Computers realisiert sein.

Dennoch wird bevorzugt, dass Parameter und/oder Ausgangsinformationen, die die Bewertungseinrichtung zur Bestimmung der Unsicherheit der Messwerte benötigt, individuell für eine bestimmte Messanordnung und/oder für einen bestimmten Probanden festgelegt werden. Ein wichtiger Parameter für die Erhöhung der Unsicherheit ist beispielsweise die maximal erwartete Änderungsrate der Messgröße. Ein weiterer Parameter ist die Unsicherheit, die bei fehlerfreier Messung der physiologischen Messgröße besteht. Sie bestimmt sich insbesondere aus der Genauigkeit des Sensors, aus äußeren Einflüssen und aus der Anordnung des Sensors am Probanden.

Anhand von Fig. 2 wird nun eine besonders bevorzugte Ausführungsform des Verfahrens zur Bestimmung der Unsicherheit beschrieben. Auf der horizontalen Achse ist in Fig. 2 die Zeit t aufgetragen, auf der vertikalen Achse der Widerstand R (die gemessene physiologische Größe bzw. der jeweilige Wert, der ausgegeben wird). Außerdem ist ohne Bezug zu der Skalierung der vertikalen Achse (als strichpunktierte Linie) die Unsicherheit der Messwerte aufgetragen. Durch eine ununterbrochene Linie ist der ausgegebene überprüfte bzw. korrigierte Messwert, d.h. der validierte Messwert RV, aufgetragen. In Zeiten, in denen der von der Sensorik erfasste und zu der Überprüfung übertragene Messwert von dem ausgegebenen validierten Messwert RV abweicht, ist der Verlauf des von der Sensorik gelieferten Wertes mit R0 und durch eine gestrichelte Linie dargestellt.

Für die Überprüfung, ob der Eingangswert R0 der Überprüfung fehlerhaft ist, werden ein Minimalwert Rmin und ein Maximalwert Rmax definiert. Rmin und Rmax bilden die Grenzen eines Wertebereiches, innerhalb dem plausible Messwerte liegen können. Beispielsweise kann ein geringerer Messwert als Rmin bei einer fehlerfreien Messung nicht vorkommen, da selbst eine sich durchgehend zwischen den Messelektroden erstreckende Schweißschicht einen größeren Widerstand hätte. Der Minimalwert Rmin kann daher von der Anordnung der Elektroden abhängen. Umgekehrt weist beispielsweise ein größerer Messwert als der Maximalwert Rmax auf ungenügenden elektrischen Kontakt der Elektroden zu der Haut des Probanden hin. Auch der Maximalwert Rmax hängt von der Messanordnung ab, insbesondere vom Abstand der Elektroden und optional von dem Hauttyp des Probanden. Ferner ist die maximal mögliche Änderungsrate des Messwertes vorgegeben. Wird die Änderungsrate überschritten, d.h. ändert sich der Messwert schneller als mit der Änderungsrate nach unten oder nach oben, wird auch das Vorliegen eines Fehlers erkannt. Beispielsweise kann eine Elektrode durch mechanische Einwirkung nicht mehr denselben guten Kontakt zu der Haut haben, wie bei dem Messwert zuvor und dadurch eine unplausible Änderung hervorgerufen werden. In der Praxis wird vorzugsweise dadurch überprüft, ob die maximal erlaubte Änderungsrate überschritten ist, dass zwei in der zeitlichen Folge der Messwerte nacheinander liegende Werte miteinander verglichen werden und dabei die dazwischen liegende Zeit berücksichtigt wird. Dabei können die zwei Messwerte unmittelbar aufeinander folgen. Wenn allerdings der ältere Messwert fehlerhaft ist, wird vorzugsweise auf noch frühere Messwerte zum Vergleich zurückgegriffen

Aus Fig. 2 ist erkennbar, dass der Messwert RV bzw. R0 zunächst bis zu dem Zeitpunkt t0 konstant verläuft. Da er innerhalb des Wertebereichs [Rmin; Rmax] liegt, wird er als fehlerfrei erkannt und unverändert als Ausgangswert RV ausgegeben. Zum Zeitpunkt t0 springt die Eingangsgröße R0 jedoch plötzlich auf einen höheren Wert. Durch einen Vergleich mit der maximal zulässigen Änderungsrate wird festgestellt, dass die Änderungsrate überschritten ist und es wird auf Fehler erkannt. Als Folge dieses Ergebnisses wird der zuletzt als fehlerfrei festgestellte Eingangswert als Ausgangswert RV ausgegeben und wird die Unsicherheit U um die maximal erlaubte Änderungsrate erhöht. Der nächste auf Fehler untersuchte Eingangswert R0 liegt ebenfalls noch auf dem hohen Niveau des vorangegangenen Eingangswertes (Zeitpunkt t1). Durch eine Berücksichtigung der Historie des Eingangswertes R0 wird ebenfalls auf Fehler erkannt und wird wiederum der zuletzt als fehlerfrei erkannte Eingangswert als Ausgangswert RV ausgegeben.

In der folgenden Zeit bis zum Zeitpunkt t2 werden wieder geringere Eingangswerte R0 geliefert und werden diese als fehlerfrei klassifiziert. Da die Historie der zuletzt ausgewerteten Eingangswerte R0 aber noch fehlerhafte Messwerte enthält, wird die Unsicherheit U nicht sofort wieder reduziert, sondern erst nachdem eine Folge von n (positive ganze Zahl größer als 1) Werten, die unmittelbar aufeinander folgen, fehlerfrei sind. Dann wird die Unsicherheit U wieder auf ihren Anfangswert reduziert. Die Geschwindigkeit der Reduktion der Unsicherheit kann dabei optional davon abhängen, wie viele der unmittelbar dem aktuellen Eingangswert vorangehenden Messwerte fehlerfrei sind.

Zum Zeitpunkt t2 wird nun festgestellt, dass der Eingangswert R0 auf einen Wert unter dem Minimalwert Rmin absinkt. Es wird daher wieder auf Fehler erkannt und der zuletzt als fehlerfrei erkannte Eingangswert wird als Ausgangswert RV ausgegeben. Da sich dieser Vorgang für die folgenden Eingangswerte R0 bis zum Zeitpunkt t3 wiederholt, wird die Unsicherheit U über den gesamten Zeitraum t2 bis t3 um die maximale Änderungsrate erhöht und erreicht einen sehr hohen Wert, d.h. die Unsicherheit des am Ende des Zeitintervalls [t2; t3] ausgegebenen Wertes RV ist sehr hoch. Ab dem Zeitpunkt t3 werden wieder gültige Messwerte erkannt, die als fehlerfrei angenommen werden können. Folglich kann die Unsicherheit U wieder erniedrigt werden.

Alle in dieser Beschreibung erwähnten Funkverbindungen können auch durch elektrisch leitende Verbindungen ersetzt werden und umgekehrt.

## Patentansprüche

1. Verfahren zur Messung einer Hauttemperatur und/oder eines elektrischen Hautwiderstandes, wobei
- ein Messwert (R0) der Hauttemperatur und/oder des elektrischen Hautwiderstandes von einem Sensor (3) erfasst wird,
- eine Unsicherheit (U) des Messwertes (R0) definiert wird, um die ein tatsächlicher Wert der Hauttemperatur und/oder des elektrischen Hautwiderstandes von dem Messwert (R0) abweichen kann,
- der Messwert (R0) in einer Überprüfung auf Plausibilität, d.h. Fehler überprüft wird, wobei bei der Überprüfung auf ein Vorliegen eines Fehlers erkannt wird, wenn der Messwert (R0) außerhalb eines vorgegebenen, erwarteten Messwertbereiches liegt, und/oder wobei bei der Überprüfung auf ein Vorlieben eines Fehlers erkannt wird, wenn die Messgröße sich schneller als mit einer vorgegebenen Änderungsrate ändert, und/oder auf ein Vorliegen eines Fehlers erkannt wird, wenn die Messgröße sich langsamer als eine vorgegebene Änderungsrate ändert,
- die Unsicherheit (U) abhängig von einem Ergebnis der Überprüfung vergrößert wird, beibehalten wird oder verkleinert wird,
- die Unsicherheit (U) angegeben wird und
- kontinuierlich ein validierter Messwert (RV) ausgegeben wird, wobei der validierte Messwert (RV) gleich dem Messwert (R0) ist, wenn bei der Überprüfung des Messwertes (R0) kein Fehler festgestellt wird, und wobei der validierte Messwert (RV) durch Korrektur des Messwertes (R0) erzeugt wird, wenn bei der Überprüfung des Messwertes (R0) ein Fehler festgestellt wird.

2. Verfahren nach dem vorhergehenden Anspruch, wobei eine Information darüber angegeben und/oder ausgegeben wird, ob ein Messwert fehlerhaft ist.

3. Verfahren nach einem der vorhergehenden Ansprüche, wobei der Sensor (3) an einer festen Position relativ zu einer Hautoberfläche eines Probanden fixiert ist.

4. Verfahren nach einem der vorhergehenden Ansprüche, wobei eine Fehlerkategorie angegeben und/oder ausgegeben wird.

5. Verfahren nach einem der vorhergehenden Ansprüche, wobei ein Grad eines Fehlers angegeben und/oder ausgegeben wird.

6. Verfahren nach einem der vorhergehenden Ansprüche, wobei die Fehler Überprüfung für eine Folge von Messwerten des Sensors (3) durchgeführt wird, wobei zumindest ein vorhergehender Messwert der Folge gespeichert wird (gespeicherter Messwert) und wobei durch einen Vergleich des gespeicherten Messwertes mit einem folgenden, zweiten Messwert eine Änderungsrate zum Vergleich mit der vorgegebenen Änderungsrate bestimmt wird.

7. Verfahren nach einem der beiden vorhergehenden Ansprüche, wobei die Unsicherheit (U) eines späteren Messwertes gegenüber der Unsicherheit (U) eines früheren Messwertes um einen Betrag erhöht wird, der abhängig von der vorgegebenen Änderungsrate und abhängig von einer Zeitdifferenz zwischen dem Messzeitpunkt des späteren Messwertes und dem Messzeitpunkt des früheren Messwertes ist.

8. Verfahren nach einem der vorhergehenden Ansprüche, wobei die Unsicherheit (U) abhängig von einer Art eines festgestellten Fehlers weniger stark verändert wird oder stärker verändert wird.

9. Verfahren nach einem der vorhergehenden Ansprüche, wobei der Sensor (3) ein nicht invasiver Sensor ist, der an einer Hautoberfläche eines Probanden angeordnet ist.

10. Verfahren nach einem der vorhergehenden Ansprüche, wobei die Fehler-Überprüfung für eine Folge von Messwerten des Sensors (3) durchgeführt wird, wobei jeweils die Unsicherheit (U) von einem vorhergehenden Messwert der Folge (vorhergehende Unsicherheit) bei einem folgenden, zweiten Messwert verwendet wird und wobei nach der Überprüfung des zweiten Messwertes die vorhergehende Unsicherheit abhängig von dem Ergebnis der Überprüfung beibehalten oder verändert wird und die beibehaltene oder veränderte Unsicherheit dem zweiten Messwert zugeordnet wird.

11. Anordnung (1) zur Messung einer Hauttemperatur und/oder eines elektrischen Hautwiderstandes, wobei die Anordnung Folgendes aufweist:
- einen Sensor (3), der ausgestaltet ist, Messwerte (R0) einer physiologischen Messgröße zu messen,
- eine Messwert-Überprüfungseinrichtung (9), die ausgestaltet ist, einen Messwert (R0) auf Plausibilität, d.h. Fehler zu überprüten,
wobei bei der Überprüfung auf ein Vorliegen eines Fehlers erkannt wird, wenn der Messwert (R0) außerhalb eines vorgegebenen, erwarteten Messwertbereiches liegt, und/oder wobei bei der Überprüfung auf ein Vorliegen eines Fehlers erkannt wird, wenn die Messgröße sich schneller als mit einer vorgegebenen Änderungsrate ändert, und/oder auf ein Vorliegen eines Fehlers erkannt wird, wenn die Messgröße sich langsamer als eine vorgegebene Änderungsrate ändert, und
- eine Bewertungseinrichtung (11), die ausgestaltet ist, eine Unsicherheit (U) für Messwerte abhängig von einem Ergebnis einer Überprüfung der Messwert-Überprüfungseinrichtung (9) zu vergrößern, beizubehalten oder zu verkleinern, wobei die Unsicherheit (U) des Messwertes (R0) angibt, wie weit ein tatsächlicher Wert der physiologischen Messgröße von dem Messwert (R0) abweichen kann, und die ausgestaltet ist, die Unsicherheit auszugeben,
wobei die Anordnung ausgestaltet ist, kontinuierlich einen validierten Messwert (RV) auszugeben, wobei der validierte Messwert (RV) gleich dem Messwert (R0) ist, wenn bei der Überprüfung des Messwertes (R0) kein Fehler festgestellt wird, und wobei der validierte Messwert (RV) ein durch Korrektur des Messwertes (R0) erzeugter Wert ist, wenn bei der Überprüfung des Messwertes (R0) ein Fehler festgestellt wird.

## Claims

1. Method for measuring a skin temperature and/or an electrical skin resistance, in which
- a measurement value (R0) of the skin temperature and/or the electrical skin resistance is acquired by a sensor (3),
- an uncertainty (U) of the measurement value (R0) is defined, by which amount an actual value of the skin temperature and/or the electrical skin resistance can deviate from the measurement value (R0),
- the measurement value (R0) is examined with regard to plausibility, that is to say for errors, in an examination, a presence of an error being detected in the examination if the measurement value (R0) lies outside a prescribed, expected measurement value range and/or a presence of an error being detected in the examination if the measurement variable is changing faster than a prescribed rate of change and/or a presence of an error being detected if the measurement variable is changing slower than a prescribed rate of change,
- the uncertainty (U) is increased, maintained or decreased depending on a result of the examination,
- the uncertainty (U) is specified, and
- a validated measurement value (RV) is continuously output, the validated measurement value (RV) equalling the measurement value (R0) if no error was determined in the examination of the measurement value (R0) and the validated measurement value (RV) being generated by correcting the measurement value (R0) if an error was determined in the examination of the measurement value (R0).

2. Method according to the preceding claim, in which information as to whether a measurement value is faulty is specified and/or output.

3. Method according to one of the preceding claims, in which the sensor (3) is attached at a fixed position relative to a skin surface of a subject.

4. Method according to one of the preceding claims, in which an error category is specified and/or output.

5. Method according to one of the preceding claims, in which a degree of error is specified and/or output.

6. Method according to one of the preceding claims, in which the error examination is carried out for a sequence of measurement values of the sensor (3), at least one preceding measurement value in the sequence being stored (stored measurement value) and a comparison of the stored measurement value and a subsequent second measurement value determining a rate of change for comparison with the predetermined rate of change.

7. Method according to one of the two preceding claims, in which the uncertainty (U) of a later measurement value is increased compared to the uncertainty (U) of an earlier measurement value by an amount which depends on the prescribed rate of change and depends on a time difference between the measurement time of the later measurement value and the measurement time of the earlier measurement value.

8. Method according to one of the preceding claims, in which the uncertainty (U) is changed to a greater or a lesser extent depending on a type of error detected.

9. Method according to one of the preceding claims, in which the sensor (3) is a non-invasive sensor which is arranged on a skin surface of a subject.

10. Method according to one of the preceding claims, in which the error examination is carried out for a sequence of measurement values of the sensor (3), the uncertainty (U) of a preceding measurement value in the sequence (preceding uncertainty) being used in each case for a subsequent second measurement value, and the preceding uncertainty being maintained or changed after the examination of the second measurement value, depending on the result of the examination, and the maintained or changed uncertainty being assigned to the second measurement value.

11. Arrangement (1) for measuring a skin temperature and/or an electrical skin resistance, the arrangement having the following:
- a sensor (3) designed to measure measurement values (R0) of a physiological measurement variable,
- a measurement-value examination device (9) designed to examine a measurement value (R0) with regard to plausibility, that is to say for errors, a presence of an error being detected in the examination if the measurement value (R0) lies outside a prescribed, expected measurement value range and/or a presence of an error being detected in the examination if the measurement variable is changing faster than a prescribed rate of change and/or a presence of an error being detected if the measurement variable is changing slower than a prescribed rate of change, and
- an evaluation device (11) designed to increase, maintain or decrease an uncertainty (U) for measurement values depending on a result of an examination of the measurement-value examination device (9), the uncertainty (U) of the measurement value (R0) specifying how far an actual value of the physiological measurement variable can deviate from the measurement value (R0), and which measurement-value evaluation device is designed to output the uncertainty,
in which the arrangement is designed to continuously output a validated measurement value (RV), the validated measurement value (RV) equalling the measurement value (R0) if no error was determined in the examination of the measurement value (R0) and the validated measurement value (RV) being a value generated by correcting the measurement value (R0) if an error was determined in the examination of the measurement value (R0)

## Revendications

1. Procédé de mesure d'une température cutanée et/ou d'une résistance cutanée électrique, dans lequel
- une valeur de mesure (R0) de la température cutanée et/ou de la résistance cutanée électrique est saisie par un capteur (3) ;
- une incertitude (U) de la valeur de mesure (R0) est définie, de laquelle une valeur réelle de la température cutanée et/ou de la résistance cutanée électrique de la valeur de mesure (R0) peut s'écarter,
- la valeur de mesure (R0) est contrôlée dans un contrôle de plausibilité, c'est-à-dire de l'erreur, le contrôle de la présence d'une erreur permettant de reconnaître si la valeur de mesure (R0) se situe en dehors d'une plage de valeurs de mesures attendue prédéterminé, et/ ou le contrôle de la présence d'une erreur permettant de reconnaître si la grandeur de mesure se modifie plus vite qu'avec un taux de modification prédéterminé et/ou permettant de reconnaître si la grandeur de mesure se modifie plus lentement qu'un taux de modification prédéterminé,
- l'incertitude (U) est accrue, conservée ou diminuée en fonction d'un résultat du contrôle,
- l'incertitude (U) est définie et
- une valeur de mesure validée (RV) est définie en continu, la valeur de mesure validée (RV) étant égale à la valeur de mesure (R0), lorsque lors du contrôle de la valeur de mesure (R0), aucune erreur n'est constatée et la valeur de mesure validée (RV) étant générée par la correction de la valeur de mesure (R0) lorsque, lors du contrôle de la valeur de mesure (R0), aucune erreur n'est constatée.

2. Procédé selon l'une des revendications précédentes, dans lequel une information est indiquée ou émise sur le fait qu'une valeur de mesure est ou non erronée.

3. Procédé selon l'une des revendications précédentes, dans lequel le capteur (3) est fixé sur une position fixe par rapport à une surface cutanée d'un sujet.

4. Procédé selon l'une des revendications précédentes, dans lequel une catégorie d'erreur est indiquée et/ ou émise.

5. Procédé selon l'une des revendications précédentes, dans lequel un degré d'erreur est indiqué et/ ou émis.

6. Procédé selon l'une des revendications précédentes, dans lequel le contrôle des erreurs est réalisée pour une série de valeurs de mesure du capteur (3), au moins une valeur de mesure précédente de la série étant enregistrée (valeur de mesure enregistrée) et par une comparaison de la valeur de mesure enregistrée avec une deuxième valeur de mesure suivante, un taux de modification est déterminé pour comparaison avec le taux de modification prédéterminé.

7. Procédé selon l'une des deux revendications précédentes, dans lequel l'incertitude (U) d'une valeur de mesure ultérieure par rapport à l'incertitude (U) d'une valeur de mesure antérieure est augmentée d'un niveau qui dépend du taux de modification prédéterminé et qui dépend d'une différence de temps entre le moment de mesure de la valeur de mesure ultérieure et le moment de mesure de la valeur de mesure antérieure.

8. Procédé selon l'une des revendications précédentes, dans lequel l'incertitude (U) est modifiée moins fortement ou est modifiée plus fortement en fonction d'un type d'une erreur constatée.

9. Procédé selon l'une des revendications précédentes, dans lequel le capteur (3) est un capteur non invasif qui est placé sur la surface cutanée d'un sujet.

10. Procédé selon l'une des revendications précédentes, dans lequel le contrôle des erreurs est réalisé pour une série de valeurs de mesure du capteur (3), dans lequel respectivement l'incertitude (U) d'une valeur de mesure prédéterminée de la série (incertitude précédente) est utilisée pour une deuxième valeur de mesure suivante et dans lequel, après le contrôle de la deuxième valeur de mesure, l'incertitude précédente est conservée ou modifiée en fonction du résultat du contrôle et l'incertitude conservée ou modifiée est affectée à la deuxième valeur de mesure.

11. Dispositif (1) de mesure d'une température cutanée et/ou d'une résistance cutanée électrique, le dispositif présentant les éléments suivants :
- un capteur (3) qui est configuré pour mesurer des valeurs de mesure (R0) d'une grandeur de mesure physiologique,
- un dispositif de contrôle de la valeur de mesure (9) qui est conçu pour contrôler la plausibilité d'une valeur de mesure (R0), c'est-à-dire contrôler les erreurs, le contrôle de la présence d'une erreur permettant de reconnaître si la valeur de mesure (R0) se situe en dehors d'une plage de valeurs de mesures attendue prédéterminée, et/ou le contrôle de la présence d'une erreur permettant de reconnaître si la grandeur de mesure se modifie plus vite qu'avec un taux de modification prédéterminé et/ou permettant de reconnaître si la grandeur de mesure se modifie plus lentement qu'un taux de modification prédéterminé,
- un dispositif d'analyse (11) qui est conçu pour agrandir, conserver ou diminuer une incertitude (U) de valeurs de mesure en fonction d'un résultat d'un contrôle du dispositif de contrôle de la valeur de mesure (9), l'incertitude (U) de la valeur de mesure (R0) indiquant dans quelle proportion une valeur réelle de la grandeur de mesure physiologique peut s'écarter de la valeur de mesure (R0) et qui est conçu pour indiquer l'incertitude,
le dispositif étant conçu pour émettre en continu une valeur de mesure validée (RV), la valeur de mesure validée (RV) étant identique à la valeur de mesure (R0) lorsque aucune erreur n'est constatée lors du contrôle de la valeur de mesure (R0) et la valeur de mesure validée (RV) étant une valeur produite par la correction de la valeur de mesure (R0) lorsque aucune erreur n'est constatée lors du contrôle de la valeur de mesure (R0).
